# EUROPEAN PATENT SPECIFICATION

(11) **EP 2 392 291 B1**
(45) Date of publication and mention of the grant of the patent: **18.12.2013**
(21) Application number: 10164565.3
(22) Date of filing: 01.06.2010
(51) Int. Cl.: A61C 13/30

(54) **Dental posts**
Dentalpfosten
Supports dentaires

(43) Date of publication of application: 07.12.2011
(73) Proprietor: Coltène/Whaledent AG, 9450 Altstaetten (CH)
(72) Inventor: Böhner, Ralf, 9451, Kriessern (CH); Mannschedel, Werner, 89129, Langenau (DE)
(74) Representative: Hepp Wenger Ryffel AG

(56) References cited:
- EP-A1- 2 172 169
- EP-A2- 1 147 748
- GB-A- 2 315 676
- US-A1- 2007 141 535
- US-A1- 2007 207 444
- US-A1- 2009 325 130

## Description

The present invention pertains to the technical field of dental restorations, especially dental posts.

Dental posts are used frequently in dental restorations. Such dental posts are placed in a suitably prepared root canal, e.g. by means of a cement, with a head portion of the post extending over the prepared tooth. This head portion of the post is the basis for restoration of the tooth.

A multitude of dental posts are available on the market today. Posts with a central filament or yarn, and a synthetic resin around this filament or yarn, are known e.g. from DE 39 01 640 A1.

It is advantageous, that dental posts be made visible to X-rays for the clinical dentist.

US6224377 B1 describes dental posts made of a radio-opaque composite. Essentially, a radio-opaque reinforcing fibre is used and further barium and zirconia glass is added to increase radio opacity of the post. This has the drawback though, that radio opacity is uniformly distributed within the post and radiographic images do not allow for a three dimensional representation and the edges of the post, which are important for the dentist to study, become blurry and look lacerated.

EP 1 147 748 A2 discloses an endocanal pin, comprising one or more layers of fibre fabric, embedded in a resinous matrix, which wind around a support core in a composite material arranged along the axis of the pin.

Accordingly, it is an object of the present invention to overcome the drawbacks of the prior art.

In particular, a dental post is provided that has an optimal radio opacity and contrast in relation to bone, enamel, dentine and cementum or gutta percha, thus allowing for a three dimensional representation of the post with X-ray.

The above object is solved by a dental post according to the independent claims.

One aspect of the present invention is a radio-opaque, multi-layered dental post with a head section and a body section, comprising:
- a core with a first material, wherein the core is made from a fibrous material which is embedded in or impregnated with said first material, and
- a coating of a second material on said core, wherein said coating comprises a thermoplastic or duroplastic material including at least one radio-opaque additive, and wherein the layer of the second material has a varying thickness in the head and body section.

The first material and the second material may be thermoplastic or duroplastic. Preferably, at least the second material is duroplastic. Most preferably, both the first and the second material are duroplastic.

The core with said first material is made from a fibrous material which is embedded in or impregnated with said first material. Long fibres are preferably used as the fibrous material.

In preferred embodiments, the fibrous material essentially extends over the whole length of the core. However, the fibrous material may also be distributed in pieces and embedded in the first material of the core.

Glass fibers (radio-opaqe or radio-transparent), aramid fibers, kevlar fibers and carbon fibers are conceivable.

In the context of the present invention, embedded can also mean the rod is impregnated with a thermoplastic or duroplastic material.

Dental post, as used herein, also comprises dental pins.

The surface texture of the post may be of any suitable size and shape aiding in handling and/or fitting/retaining in the root canal. Typical surface textures are e.g. lengthwise grooves, e.g. parallel to or wound around the central axis of the post, interconnected or isolated; circumferential grooves, either at a right angle to the central axis of the post or tilted to central axis of the post, interconnected or isolated; undercuts. The surface texture can either be formed as part of the manufacturing process or be formed after the actual manufacturing in a refinement step.

In the context of the present invention, radio-opacque, radio-opacity and values for radio-opacity are to be construed as defined by ISO 4049:2000(E).

According to a preferred embodiment the dental post is of substantially cylindrical or conical shape, preferably of substantially circular-cylindrical, circular-conical or substantially elliptic-cylindrical shape.

In a preferred embodiment, the long fiber forms a central filament or yarn. The diameter of the central filament(s) and/or yarn(s) typically is in the range of about 0,3 mm to about 2,5 mm, more preferably in the range of about 0,5 mm to about 2,0 mm. In most cases, the purpose of the central filament is to provide sufficient strength for the dental post. Therefore, the diameter of the central filament will be dictated in each case by the desired properties of the post employed. The person of routine skill in the art will readily realize necessary diameters, based on these considerations and, if necessary, routine experiments.

Moreover, a multitude, e.g. a bundle of filament(s) and/or yarn(s) can be used. For example, further filaments and/or fibers can be wound around a central filament or yarn, as is generally known in the art (cf e.g. DE 39 01 640 A1.) Also braided fibers and/or filaments can be used in the context of the present invention, such as e.g. disclosed in US 6,402,519 B1. )

In further embodiments of the present invention, the material of the filament(s) and/or yarn(s) is/are chosen from the group consisting ceramic; carbon; graphite; Alumina/Silica/Boria composites such as Nextel® 312, Nextel® 440; Al₂O₃; quartz; glass such as e.g. alkali resistant glass (preferably comprising ZrO₂), glass with high tensile strength (e.g. S-2 glass); SiO₂; Kevlar; metal; plastics such as e.g. acrylonitrile butadiene styrene (ABS), polyamide (PA), polyacrylates such as e.g. polymethylmethacrylate (PMMA) and polymethacrylate (PMA), polyetherketones (PEK), polyetheretherketones (PEEK), polysulfones (PS), polyethersulfones (PES), polyphenylenes, Polycarbonate (PC), polyethylene terephthalat (PET), polyethylene (PE), polypropylene (PP), polystyrene (PS), Polyvinylchloride (PVC); Liquid Crystal Polymer (LCP), Polyacetal (POM or Acetal), Polyacrylonitrile (PAN or Acrylonitrile), Polyamide-imide (PAI), Polyaryletherketone (PAEK or Ketone), Polybutylene terephthalate (PBT), Polycaprolactone (PCL), Polychlorotrifluoroethylene (PCTFE), Polyethylene terephthalate (PET), Polycyclohexylene dimethylene terephthalate (PCT), Polyhydroxyalkanoates (PHAs), Polyketone (PK), Polyester, Polyetherimide (PEI), Polyethylenechlorinates (PEC), Polyimide (PI), Polylactic acid (PLA), Polymethylpentene (PMP), Polyphenylene oxide (PPO), Polyphenylene sulfide (PPS), Polyphthalamide (PPA), Polystyrene (PS), Polysulfone (PSU), Polyurethane (PU), Polyvinyl acetate (PVA), Polyvinyl chloride (PVC), Polyvinylidene chloride (PVDC), Styrene-acrylonitrile (SAN); and combinations thereof.

The types of fibreglass used in the art are, for example, mainly E-glass (aluminium-borosilicate glass with less than 1 wt.% alkali oxides, mainly used for glass-reinforced plastics), but also A-glass (alkali-lime glass with little or no boron oxide), E-CR-glass (alumino-lime silicate with less than 1 wt.% alkali oxides with high acid resistance), C-glass (alkali-lime glass with high boron oxide content), D-glass (borosilicate glass with high dielectric constant), R-glass (alumino silicate glass without MgO and CaO with high mechanical requirements), and S-glass (alumino silicate glass without CaO but with high MgO content with high tensile strength). For a person skilled in the art the choice of fiber glass is easily conceivable for the intended application and can be referenced, for instance, from "Fibers, 5. Synthetic Inorganic" (Fitzer, E., et al., Ullmann's Encyclopedia of Industrial Chemistry, Wiley VCH Verlag GmbH & Co. KGaA, Weinheim, Germany).

An illustrative example of a core within the scope of the invention comprising a first material and at least one further radio opaque additive is described in EP 793,474 B1. The post comprising said core comprises a further coating of a second material on said core, wherein said coating comprises at least one radio-opaque additive.

Additionally, zirconium dioxide (zirconia) or barium glass can be incorporated into the filament(s) or fiber(s) in order to achieve radio-opacity, as is known from e.g. EP 992 223 B1.

Suitable radio-opaque additives for both the first and the second material are well known in the art of dentistry, and can be chosen with ease by the person of routine skill in the art. Heavy metal oxides, halogenides, sulfates, oxyhalogenides comprising a heavy metal element having an atom number greater than 29 can be used. Oxides and/or salts of Zirconium (Zr), Barium (Ba), Strontium (Sr), Titanium (Ti), Bismouth (Bi) and Zinc (Zn) are preferred, as well as fluorides and/or oxides of Ytterbium (YbF₃, Yb₂O₃) and Yttrium. Dental glass (for example dental barium glass) can also be used in this respect, especially further comprising at least one of the aforementioned oxides or fluorides. In essence, all fluorides or oxides of "rare earth" elements are known to impart radio-opacity for dental applications (as disclosed in EP 189540 B1). Therefore further conceivable radio-opaque additives can be fluorides of lanthanum, cerium, samarium and fluorides or oxides of gadolinium, dysprosium and/or erbium. Most preferred are ZrO₂, BaSO₄, YbF₃, BiOCl and YF₃ .

The first and/or the second material can be thermally, chemically or light-polymerizable resins. Examples are epoxy-, acrylate-, polyester-, vinylester- or phenolic resins. Hardening can be radically, cationic or by polycondensation or polyaddition. The first and/or second material can further comprise additives that aid the respective hardening process, such as, for example, photoinitiators.

In a preferred embodiment, the radio-opaque additive to the resin coating is a metal oxide.

In a particular embodiment, the radio-opaque additive to the resin coating is a metal fluoride.

In a particular embodiment, thermoplastic material(s) of the coating and/or the core is/are chosen from the group consisting of acrylonitrile butadiene styrene (ABS), polyamide (PA), polyacrylates such as e.g. polymethylmethacrylate (PMMA) and polymethacrylate (PMA), polyetherketones (PEK), polyetheretherketones (PEEK), polysulfones (PS), polyethersulfones (PES), polyphenylenes, Polycarbonate (PC), polyethylene terephthalat (PET), polyethylene (PE), polypropylene (PP), polystyrene (PS), Polyvinylchloride (PVC); Liquid Crystal Polymer (LCP), Polyacetal (POM or Acetal), Polyacrylonitrile (PAN or Acrylonitrile), Polyamide-imide (PAI), Polyaryletherketone (PAEK or Ketone), Polybutylene terephthalate (PBT), Polycaprolactone (PCL), Polychlorotrifluoroethylene (PCTFE), Polyethylene terephthalate (PET), Polycyclohexylene dimethylene terephthalate (PCT), Polyhydroxyalkanoates (PHAs), Polyketone (PK), Polyester, Polyetherimide (PEI), Polyethylenechlorinates (PEC), Polyimide (PI), Polylactic acid (PLA), Polymethylpentene (PMP), Polyphenylene oxide (PPO), Polyphenylene sulfide (PPS), Polyphthalamide (PPA), Polystyrene (PS), Polysulfone (PSU), Polyurethane (PU), Polyvinyl acetate (PVA), Polyvinyl chloride (PVC), Polyvinylidene chloride (PVDC), Styrene-acrylonitrile (SAN), and mixtures thereof.

In a particular embodiment, duroplastic material(s) of the core are chosen from the group consisting of epoxy resins, polyurethanes (PUR), acrylates, methacrylates, polyesters and/or vinylesters.

The choice of the resin can ultimately be adapted to increase compatibility with the cement, for example radically polymerizationable resin monomers for methacrylate cements.

In a particular embodiment, the post is made of a fiber or filament which is radio-opaque (preferably glass containing zirconium dioxide in order to allow for radio-opacity) enwrapped with thermoplastic material, wherein the difference between the refraction index of the fiber or filament and the thermoplastic/duroplastic material is less than 0,15. This allows for providing radio-opaque posts that are transparent for the unaided eye, as is discussed in any detail in EP 1 115 349 B1 .

Any desired surface texture can be produced by directed thermal shaping and/or re-shaping of thermoplastic material.

A thickness of the layer of the second material is in the range of about 0,01 mm to about 1,0 mm, preferably about 0,03 to about 0,7 mm, most preferably about 0,05 to about 0,5 mm.

In particular embodiments, the coating of the second material on said core comprises at least two, three or more radio-opaque additives.

In preferred embodiments, a dental post according to the present invention has the following dimensions:
- a length in the range of about 5 mm to about 40 mm, preferably about 8 to about 30 mm, most preferably about 10 to about 25 mm;
- a maximum cross-sectional diameter in the range of about 0,5 mm to about 3 mm, preferably about 0,7 to about 2,5 mm, most preferably about 0,8 to about 2,0 mm.

The dental post according to the invention has a head section and a bottom or body section. The head section is used for stabilizing the subsequent dental restoration, whereas the body section extends into the root canal.

According to the invention, the layer of the second material has a varying thickness in the head and body section. Preferably the head section of the resin coatings have a coating thickness from about 0.02 to about 1.5 mm, preferably from about 0.05 mm to about 1.2 mm and the coating thickness in the body section is from about 0.02 mm to about 0.7 mm, preferably from about 0.05 mm to about 0.5 mm.

In a further preferred embodiment, the coating thickness of at least one of the second material is in the head section from about 0.1 to about 1 mm and the coating thickness in the body section is from about 0.1 mm to about 0.3 mm.

The head and body section can be separated by a circumferential groove, for example.

In a further preferred embodiment, the dental post comprises a filament or yarn in its centre. The central filament or yarn is coated with the first material - a synthetic resin as described in the previous embodiments around this filament or yarn. Hereby casting techniques can be applied. The central yarn can be incorporated as described in DE 3901640 A1.

Yet another aspect of the present invention is a method for the production of a dental post with at least one resin coating, at least one of the resin coatings comprising radio-opaque additives. A core with a first material is provided in a casting form; and then a layer of a second material is casted around said core. Or a second material is provided in the casting form and a first material is inserted in the casting form. If photopolymerization of the second material is desired, a suitably transparent casting form can be chosen. Split casting forms can be used in order to allow for removal of the post from the mould. Alternatively, one-piece forms can be used if it is sufficiently flexible to allow for removal of the post. If heat or chemical polymerization of the second material is desired split casting forms can be used in order to allow the removal of the post from the mould. Alternatively, one-piece forms can be used, in particular those sufficiently flexible to allow for removal of the post. Of course, a lengthy object can be casted in suitable forms, and this lengthy object can then be cut down to multiple posts.

Curing can be performed thermally, chemically or by radiation means (light, electron beam). Large scale production methods can easily be adapted by persons skilled in the art, comprising split mould or elastic moulds for thermal or chemical curing and/or transparent split mould or transparent elastic mould for light curing methods.

One further aspect of the present invention is the upgrade of a conventional dental post (be it already radio-opaque or not) by coating the post with an additional layer of a second material with at least one radio-opaque additive.

Most interesting, the additional layer of the second material with at least one radio-opaque additive emphasizes the edges of the post in x-ray photographs, instead of conventional radio-opaque posts that often tend to blur at the outer margin in x-ray photographs.

This allows for upgrading the radio-opacity of otherwise only insubstantially radio-opaque posts to a radio opacity providing significant advantages for the practitioner. The dental post has an optimal radio opacity and contrast in relation to bone, enamel, dentine and cementum or gutta percha, thus allowing for a three dimensional representation of the implant with X-ray.

The invention will now be described in even more detail, by means of preferred embodiments, without however limiting the scope of the invention to these embodiments. The figures schematically show:
**Fig. 1****:** schematic drawing of a tooth;
**Fig. 2a****:** schematic drawing of a dental post
**Fig. 2b****:** schematic drawing of a dental post
Fig. 2c: schematic drawing of a dental post
Fig. 2d: schematic drawing of a dental post
Fig. 2e: schematic drawing of a dental post
Fig. 2f: schematic drawing of a dental post
**Fig. 3****:** X-ray of dental posts

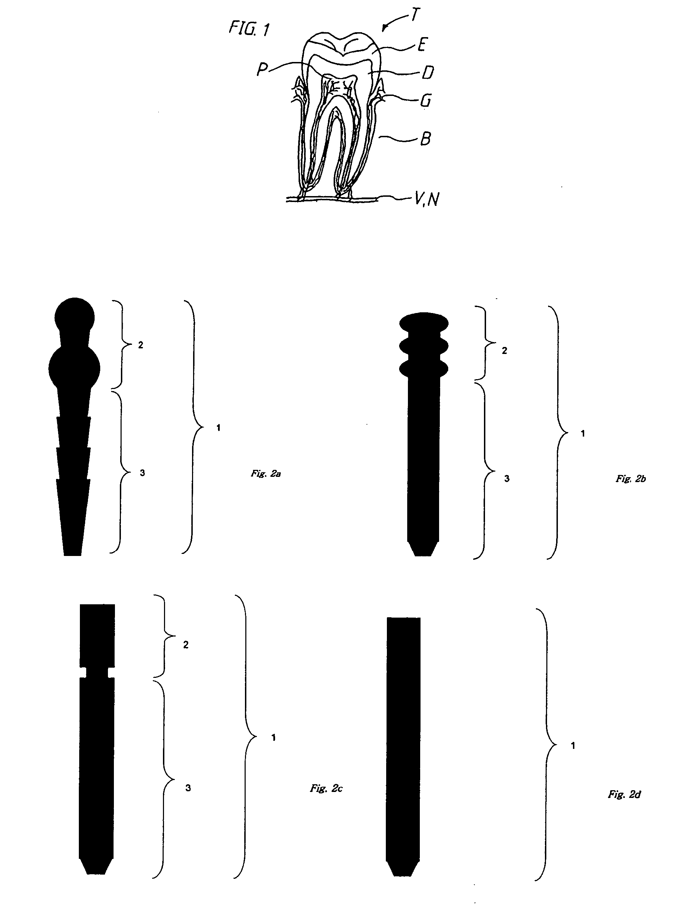

Fig. 1 schematically illustrates a tooth T. The outer layer of the tooth is called enamel E, the layer underneath is commonly referred to as dentin D. The most inner part of the tooth is the pulp P that also extends into the root canal. The pulp comprises blood vessels V and nerves N that reach through the root. The tooth T is moreover embedded into gum G and the bone B.

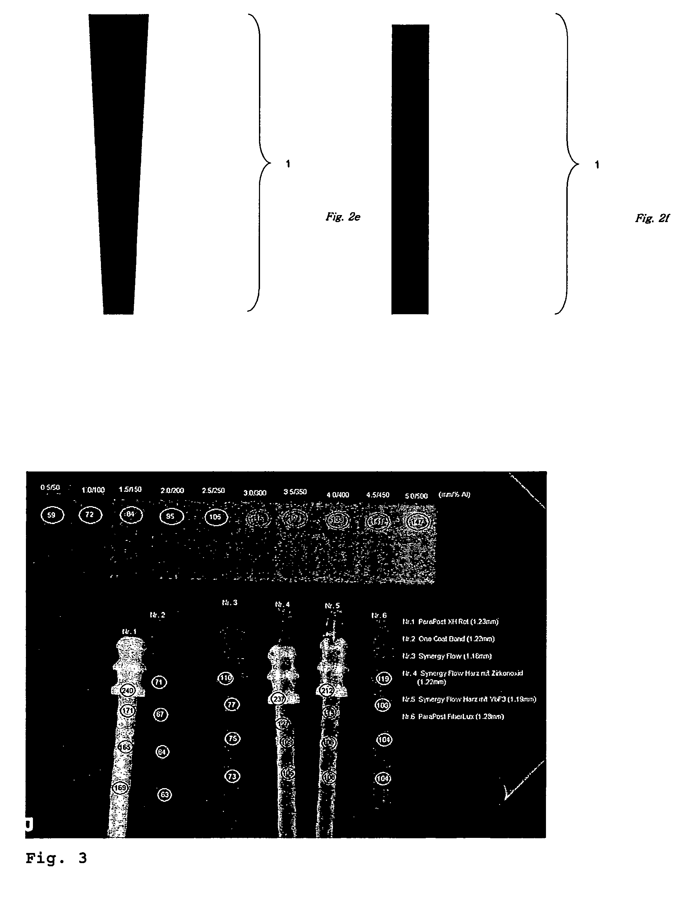

Fig.2a to 2f schematically depict alternative post fabrications. The post of Fig. 2a shows a dental post 1 with a head section 2 and a body or bottom section 3. In this particular embodiment, the head section 2 consists of two essentially spherical knobs. The body section 3 has horizontal recesses (grooves) along its surface. Fig. 2b shows an alternative dental post 1 with a head section 2 and a body section 3, whereby the head section 2 also comprises spherical protrusions that extend circumferentially around the longitudinal axis of the post 1. The body section 3 is essentially smooth, with a narrowing, terminal, essentially trapezoid bottom edge. Fig. 2c depicts a further alternative post 1, where a head 2 and body section 3 are separated by a circumferential groove. Fig. 2d depicts a further alternative post 1, with no visible head or body section. Fig. 2e depicts a further alternative post 1 with an essentially conical shape. Fig. 2f depicts a further alternative post 1 in its most basic embodiment, an essentially smooth rod.

Fig. 3 is a radiograph of different posts depicting the varying degrees of radio-opacity, made from various second materials according to the invention. Radiographs were taken with a Sirona Heliodent DS (60-kV, 7 mA).

The posts according to the invention are coated with a coating as follows:

| | |
|---|---|
| **No. 4:** | 30 % light cured dental resin |
| | 40 % dental Ba-glass |
| | 30 % Zr0₂ |
| **No. 5:** | 30 % light cured dental resin |
| | 40 % dental Ba-glass |
| | 30 % YbF₃ |

The light cured dental resin used in this example consists of > 97% dimethylacrylates (for example Bis-GMA(Bisphenol-A-glycidmethacrylate), TEGDMA (triethyleneglycoldimethlyacrylate) and others) as well as photo initiators and stabilizers. Vinyl compositions suitable for polymerisation are known to the skilled artisan and can be derived, for example, from EP 0189540 B1.

Dental Ba-glass is easily economically available. Suitable dental glass can be obtained by SCHOTT Electronic Packaging GmbH, Landshut (DE). Dental Ba-Glass herein used: Schott 8235. Alternatively Schott Dental Barium Glass G018-186, G018-053, GM27884 or GM39923 are also suitable.

Post no. 6 is grinded out of an endless fiber reinforced stick, ParaPost Fiber Lux which represents the state of the art of post used especially for good visibility in radiographs (radiopacity). Samples 2, 3, 4 and 5 were prepared with a commercially available endless fiber reinforced stick as first material (core), Indore 0.8mm HC SDI/08-09/36 OS-53, with poor visibility in radiographs. The second material (coating) of post no. 2 is One Coat® Bond. The second material (coating) of post no. 3 Synergy® Flow (63% dental Ba-glass and 27 % light cured dental resin).

Posts no. 4 and post no. 5 are posts coated with a resin comprising a radio-opaque additive, according to the invention. As can bee seen from Fig. 3, the contrast especially at the margin of the posts no.4 and 5 is improved. An Aluminium bar serves as reference ranging from 0.5 mm to 5 mm thickness thus defining an opacity scale of 50% to 500% Aluminium. Further a comparison post no. 1 is shown, which is ParaPost® XH^{™}, made of titanium.

Post no. 4 is a post according to the present invention, which comprises a further resin coating with zirconium oxide. Post no. 5 is another post according to the present invention with a further resin coating comprising Ytterbium Fluoride. As can be seen from Fig. 3, the contrast especially at the margin of the posts no. 4 and 5 is improved over the comparative examples.

Overall thickness of the post is measured at the body.

| **Post #** | **Thickness** |
|---|---|
| 1 | 1.23 mm |
| 2 | 1.22 mm |
| 3 | 1.18 mm |
| 4 | 1.22 mm |
| 5 | 1.19 mm |
| 6 | 1.26 mm |

The radio-opacity was measured and evaluated by computer software in respect to an Aluminium scale. The comparison was performed by calculating a relative radioopacity to Aluminium depending on the acceleration voltage of the x-ray generator. Radio-opacity was determined in accordance with the standardized protocol of ISO 4049:2000 (Paragraph 5 in accordance with 7.14.2, 7.14.3 and 7.14.4).

Measurement points were taken at different spots of the post, in particular the head sections (with respective protrusions), the neck (if applicable) and several spots on the bottom/body sections.

The posts for testing purposes were produced by using a casting mold made from AccuTrans transparent of the ParaPost XH titanium post. Glass fibers were cut to appropriate sizes and dye casted with the monomer filling with a syringe.

## Claims

1. A radio-opaque, multi-layered dental post with a head section and a body section, comprising:
- a core with a first material, wherein the core is made from a fibrous material which is embedded in or impregnated with said first material, and
- a coating of a second material on said core, wherein said coating comprises a thermoplastic or duroplastic material including at least one radio-opaque additive, and wherein the layer of the second material has a varying thickness in the head and body section.

2. The dental post of claim 1, wherein the first material is thermoplastic or duroplastic.

3. The dental post of claim 1 or 2, wherein the fibrous material is long fibres.

4. The dental post of claims 1 to 3, wherein the fibrous material essentially extends over the whole length of the core.

5. The dental post of claim 1 or 2, wherein the fibrous material is embedded in the first material of the core.

6. The dental post of anyone of claims 1 to 5, wherein the first material comprises at least one radio-opaque additive.

7. The dental post of anyone of claims 1 to 6, with the following dimensions:
- a length in the range of about 5 mm to about 40 mm, preferably about 8 mm to about 30 mm, most preferably about 10 mm to about 25 mm;
- a maximum cross-sectional diameter in the range of about 0.5 mm to about 3 mm, preferably about 0.7 mm to about 2.5 mm, most preferably about 0.8 mm to about 2.0 mm.

8. The dental post according to anyone of claims 1 to 7,
wherein the radio opaque additive of said first and/or second material is at least one metal oxide, preferably an oxide of an element chosen from the group consisting of zirconium, barium, strontium, titanium, bismuth and zinc.

9. The dental post according to anyone of claims 1 to 7,
wherein the radio opaque additive is a metal fluoride, preferably a fluoride of ytterbium and/or yttrium.

10. The dental post according to anyone of claims 1 to 9,
wherein the dental post is made up of a head (2) and a body (3) section, each section having a different layer thickness of the first material and the second material.

11. The dental post according to claim 10, wherein the thickness of at least one of the first material or the second material in the head (2) section is from about 0.02 to about 1.5 mm, preferably from about 0.05 mm to about 1.2 mm, most preferably from about 0.1 to about 1 mm; and the thickness of at least one of the first material or the second material in the body (3) section is from about 0.02 mm to about 0.7 mm, preferably from about 0.05 mm to about 0.5 mm, most preferably from about 0.1 mm to about 0.3 mm.

12. The dental post according to anyone of claims 1 to 11,
wherein the post comprises a filament or yarn in its centre.

13. Method for the production of a dental post with a head section and a body section, according to anyone of claims 1 to 11, comprising the steps of:
- providing a core with a first material, wherein the core is made from a fibrous material which is embedded in or impregnated with said first material, in a casting form;
- casting a layer of a second, thermoplastic or duroplastic material including at least one radio-opaque additive around said core with a varying thickness in the head
and body section.

14. Method of upgrading a insubstantially radio-opaque dental post with a head section and a body section, wherein the dental post is coated with a layer of a thermoplastic or duroplastic material comprising at least one radio-opaque additive, and wherein said layer
has a varying thickness in the head and
body section.

## Patentansprüche

1. Röntgendichter mehrschichtiger Zahnstift mit einem Kopfabschnitt und einem Körperabschnitt, umfassend:
- einen Kern mit einem ersten Material, wobei der Kern aus einem faserartigen Material besteht, das in dem ersten Material eingebettet oder damit imprägniert ist, und
- einen Überzug aus einem zweiten Material auf dem Kern, wobei der Überzug ein thermoplastisches oder duroplastisches Material umfasst, einschließlich wenigstens eines röntgendichten Zusatzstoffs, und wobei die Schicht des zweiten Materials eine variierende Dicke in dem Kopf- und dem Körperabschnitt aufweist.

2. Zahnstift gemäß Anspruch 1, wobei das erste Material thermoplastisch oder duroplastisch ist.

3. Zahnstift gemäß Anspruch 1 oder 2, wobei es sich bei dem faserartigen Material um lange Fasern handelt.

4. Zahnstift gemäß Ansprüchen 1 bis 3, wobei sich das faserartige Material im Wesentlichen über die gesamte Länge des Kerns erstreckt.

5. Zahnstift gemäß Anspruch 1 oder 2, wobei das faserartige Material in dem ersten Material des Kerns eingebettet ist.

6. Zahnstift gemäß einem der Ansprüche 1 bis 5, wobei das erste Material wenigstens einen röntgendichten Zusatzstoff umfasst.

7. Zahnstift gemäß einem der Ansprüche 1 bis 6 mit folgenden Abmessungen:
- einer Länge im Bereich von etwa 5 mm bis etwa 40 mm, vorzugsweise etwa 8 mm bis etwa 30 mm, höchst bevorzugt etwa 10 mm bis etwa 25 mm;
- einem maximalen Querschnittdurchmesser im Bereich von etwa 0,5 mm bis etwa 3 mm, vorzugsweise etwa 0,7 mm bis etwa 2,5 mm, höchst bevorzugt etwa 0,8 mm bis etwa 2,0 mm.

8. Zahnstift gemäß einem der Ansprüche 1 bis 7, wobei der röntgendichte Zusatzstoff des ersten und/oder des zweiten Materials wenigstens ein Metalloxid ist, vorzugsweise ein Oxid eines Elements ausgewählt aus der Gruppe bestehend aus zirkon, Barium, Strontium, Titan, Bismut und Zink.

9. Zahnstift gemäß einem der Ansprüche 1 bis 7, wobei der röntgendichte Zusatzstoff ein Metallfluorid ist, vorzugsweise ein Fluorid von Ytterbium und/oder Yttrium.

10. Zahnstift gemäß einem der Ansprüche 1 bis 9, wobei der Zahnstift aus einem Kopf- (2) und einem Körperabschnitt (3) besteht, wobei jeder Abschnitt eine andere Schichtdicke des ersten Materials und des zweiten Materials aufweist.

11. Zahnstift gemäß Anspruch 10, wobei die Dicke von wenigstens einem von dem ersten Material oder dem zweiten Material in dem Kopfabschnitt (2) von etwa 0,02 bis etwa 1,5 mm beträgt, vorzugsweise von etwa 0,05 mm bis etwa 1,2 mm, höchst bevorzugt von etwa 0,1 bis etwa 1 mm; und die Dicke von wenigstens einem von dem ersten Material oder dem zweiten Material in dem Körperabschnitt (3) von etwa 0,02 mm bis etwa 0,7 mm beträgt, vorzugsweise von etwa 0,05 mm bis etwa 0,5 mm, höchst bevorzugt von etwa 0,1 mm bis etwa 0,3 mm.

12. Zahnstift gemäß einem der Ansprüche 1 bis 11, wobei der Stift ein Filament oder einen Faden in seiner Mitte umfasst.

13. verfahren zum Herstellen eines Zahnstifts mit einem Kopfabschnitt und einem Körperabschnitt gemäß einem der Ansprüche 1 bis 11, umfassend die Schritte:
- Bereitstellen eines Kerns mit einem ersten Material, wobei der Kern aus einem faserartigen Material besteht, das in dem ersten Material eingebettet oder damit imprägniert ist, in einer Gussform;
- Gießen einer Schicht eines zweiten, thermoplastischen oder duroplastischen Materials, das wenigstens einen röntgendichten Zusatzstoff enthält, um den Kern, mit variierender Dicke in dem Kopf- und dem Körperabschnitt.

14. Verfahren zum Aufwerten eines unwesentlich röntgendichten Zahnstifts, der einen Kopfabschnitt und einem Körperabschnitt aufweist, wobei der Zahnstift mit einer Schicht aus einem thermoplastischen oder duroplastischen Material überzogen wird, das wenigstens einen röntgendichten Zusatzstoff enthält, und wobei die Schicht eine variierende Dicke im Kopf- und im Körperabschnitt aufweist.

## Revendications

1. Support dentaire multicouche, radio-opaque, avec une section de tête et une section de corps, comprenant
- un noyau avec un premier matériau, dans lequel le noyau est constitué d'un matériau fibreux qui est noyé dans ou imprégné avec ledit premier matériau, et
- un revêtement en un second matériau sur ledit noyau, dans lequel ledit revêtement comprend un matériau thermoplastique ou thermodurcissable comportant au moins un additif radio-opaque, et dans lequel la couche du second matériau a une épaisseur variable dans la section de tête et la section de corps.

2. Support dentaire selon la revendication 1, dans lequel le premier matériau est thermoplastique ou thermodurcissable.

3. Support dentaire selon la revendication 1 ou 2, dans lequel le matériau fibreux est formé de fibres longues.

4. Support dentaire selon revendications 1 à 3, dans lequel le matériau fibreux s'étend essentiellement sur toute la longueur du noyau.

5. Support dentaire selon la revendication 1 ou 2, dans lequel le matériau fibreux est noyé dans le premier matériau du noyau.

6. Support dentaire selon l'une quelconque des revendications 1 à 5, dans lequel le premier matériau comprend au moins un additif radio-opaque.

7. Support dentaire selon l'une quelconque des revendications 1 à 6, présentant les dimensions suivantes:
- une longueur dans la plage d'environ 5 mm à environ 40 mm, de préférence d'environ 8 mm à environ 30 mm, et de préférence encore d'environ 10 mm à environ 25 mm;
- un diamètre de section transversale maximum dans la plage d'environ 0,5 mm à environ 3 mm, de préférence d'environ 0,7 mm à environ 2,5 mm, et de préférence encore d'environ 0,8 mm à environ 2,0 mm.

8. Support dentaire selon l'une quelconque des revendications 1 à 7, dans lequel l'additif radio-opaque dudit premier et/ou second matériau est au moins un oxyde de métal, de préférence un oxyde d'un élément choisi dans le groupe composé du zirconium, du baryum, du strontium, du titane, du bismuth et du zinc.

9. Support dentaire selon l'une quelconque des revendications 1 à 7, dans lequel l'additif radio-opaque est un fluorure de métal, de préférence un fluorure d'ytterbium et/ou d'yttrium.

10. Support dentaire selon l'une quelconque des revendications 1 à 9, dans lequel le support dentaire est constitué d'une section de tête (2) et d'une section de corps (3), chaque section ayant une épaisseur de couche différente du premier matériau et du second matériau.

11. Support dentaire selon la revendication 10, dans lequel l'épaisseur d'au moins un du premier matériau ou du second matériau dans la section de tête (2) est comprise entre environ 0,02 mm et environ 1,5 mm, de préférence entre environ 0,05 mm et environ 1,2 mm, de préférence encore entre environ 0,1 mm et environ 1 mm; et l'épaisseur d'au moins un du premier matériau ou du second matériau dans la section de corps (3) est comprise entre environ 0,02 mm et environ 0,7 mm, de préférence entre environ 0,05 mm et environ 0,5 mm, et de préférence encore entre environ 0,1 mm et environ 0,3 mm.

12. Support dentaire selon l'une quelconque des revendications 1 à 11, dans lequel le support comprend un filament ou un fil en son centre.

13. Procédé de production d'un support dentaire avec une section de tête et une section de corps selon l'une quelconque des revendications 1 à 11, comprenant les étapes suivantes:
- placer un noyau avec un premier matériau, dans lequel le noyau est constitué d'un matériau fibreux qui est noyé dans ou imprégné avec ledit premier matériau, dans un moule de coulée;
- couler une couche d'un second matériau thermoplastique ou thermodurcissable comportant au moins un additif radio-opaque autour dudit noyau, avec une épaisseur variable dans la section de tête et la section de corps.

14. Procédé de amélioration d'un support dentaire non essentiellement radio-opaque avec une section de tête et une section de corps, dans lequel le support dentaire est revêtu d'une couche d'un matériau thermoplastique ou thermodurcissable comprenant au moins un additif radio-opaque, et dans lequel ladite couche a une épaisseur variable dans la section de tête et la section de corps.
